# EUROPEAN PATENT APPLICATION

(11) **EP 4 283 294 A1**
(43) Date of publication of application: **29.11.2023**
(21) Application number: 22174867.6
(22) Date of filing: 23.05.2022
(51) Int. Cl.: G01N 33/50

(54) **METHOD AND SYSTEM FOR INDICATING THE SUITABILITY OF ADMINISTERING A LABOR STIMULATING DRUG**

(71) Applicant: ObsteCare AB, 171 65 Solna (SE)
(72) Inventor: LUNDBLAD, BO, SE111 37 Stockholm (SE)
(74) Representative: Brann AB

(57) **Abstract**

A method for indicating the suitability of administering a labor stimulating drug to a pregnant woman is provided comprising the steps of: i. determining an amniotic fluid lactate concentration value, ii. comparing the amniotic fluid lactate concentration value with the set value of 10.1 mmol/l and the set value 12.0 mmol/l, whereby: a. an amniotic fluid lactate concentration value at or below 10.1 mmol/l indicates that the labor stimulating drug is suitable to be administered to the pregnant woman, and b. an amniotic fluid lactate concentration value at or above 12.0 mmol/l indicates that the labor stimulating drug is not suitable to be administered to the pregnant woman.

## Description

### Technical field

The technology proposed herein relates generally to the field of administration of labour inducing drugs and in particular to methods and systems for indicating the suitability of administering a labor stimulating drug.

### Background

One challenge in today's delivery methods is that women who have a protracted labor have a need to get help to stimulate the delivering process. This is mainly done with a drip containing synthetic oxytocin today. In some cases the uterus may be exhausted, in which case it is not susceptible to synthetic oxytocin.

If the pregnant woman has an exhausted uterus, there is an increased risk that operative methods such as vacuum, forceps or a caesarean have to be used to deliver the baby.

Different control methods are available to control that the fetus does not suffer from oxygen deficiency. Fetal blood lactate level can be measured but does not correlate to lactate level the uterus.

WO2005034762A1 discloses a method for monitoring a childbirth process of a pregnant woman. In the method, a lactate concentration of vaginal fluids is measured. If the measured lactate concentration is greater than a predetermined lactate concentration, this indicates that amniotic fluid has passed from an amnion of the pregnant woman. If the measured lactate concentration is less than a lactate threshold interval, the pregnant woman can be helped by a labor stimulating drug.

Despite the advances taught by WO2005034762A1, there remains a need to more accurately obtain an indication for when a labor stimulating drug should be administered to the pregnant woman, as well as when such administration should be avoided.

### Objectives

A primary objective of the technology proposed herein is to provide a method for indicating the suitability of administering a labor stimulating drug.

A second objective of the technology proposed herein is to provide a system for indicating the suitability of administering a labor stimulating drug.

A third objective of the technology proposed herein is to provide a sensor for measuring an amniotic fluid lactate concentration value.

A fourth objective of the technology proposed herein is to provide a method for administering a labor stimulating drug to a pregnant woman.

A fifth objective of the technology proposed herein is to provide a method for treating a pregnant woman.

### Summary

At least one of the abovementioned objectives or at least one of the further objectives which will become evident from the below description, is according to a first aspect of the technology proposed herein achieved by a method for indicating the suitability of administering a labor stimulating drug to a pregnant woman comprising the steps of:
i. determining an amniotic fluid lactate concentration value,
ii. comparing the amniotic fluid lactate concentration value with the set value of 10.1 mmol/l and the set value 12.0 mmol/l, whereby:
   a. an amniotic fluid lactate concentration value at or below 10.1 mmol/l indicates that the labor stimulating drug is suitable to be administered to the pregnant woman, and
   b. an amniotic fluid lactate concentration value at or above 12.0 mmol/l indicates that the labor stimulating drug is not suitable to be administered to the pregnant woman.

At least one of the abovementioned objectives or at least one of the further objectives which will become evident from the below description, is further, according to a corresponding second aspect of the technology proposed herein, achieved by a system for indicating the suitability of the administering of a labor stimulating drug to a pregnant woman comprising:
i. a sensor configured to determine an amniotic fluid lactate concentration value,
ii. a comparison unit configured to compare the amniotic fluid lactate concentration value with the set value of 10.1 mmol/l and the set value 12.0 mmol/l,
iii. an indication unit configured to output:
   a. an indication that the labor stimulating drug is suitable to be administered to the pregnant woman when the amniotic fluid lactate concentration value is at or below 10.1 mmol/l, and
   b. an indication that the labor stimulating drug is not suitable to be administered to the pregnant woman when the amniotic fluid lactate concentration value is at or above 12.0 mmol/l.

Accordingly, the technology proposed herein is based on the discovery that the predetermined lactate concentration set values of 10.1 mmol/l and 12.0 mmol/l may be used as reference to indicate the suitability of administering a labor stimulating drug to a pregnant woman.

The technology proposed herein thus offers several advantages. First, the technology provides for a simple, straight forward approach for determining the suitability of administering a labor stimulating drug. The technology aims to ensure safer use of labor stimulating drugs during labor, both for the woman and the fetus. Longtermly, this may decrease tokophobia and therefore result in a decreased amount of labor interventions such as the use of vacuum, forceps or a caesarean to deliver the baby. Additionally, the indication of suitability provides useful information for decision regarding the next step during labor. In other words, the woman avoids overdosage of the labor stimulating drug. Thereby, the woman is spared from unnecessary pain. Moreover, the risks for the woman associated with a protracted labor might be avoided. Additionally, subjecting of the fetus to the risks associated with a protracted labor is avoided. Further, the risks associated with labor stimulating drugs, such as uterine rupture or fetal distress due to uterine hypertony, are avoided.

Today, the healthcare staff do not have a technology for determining when a labor stimulating drug should be administered. Therefore, the labor stimulating drug might not give the expected effect. The proposed technology provides for determining when a labor stimulating drug is suitable to be administered, and when other actions should be performed. This decreases the risk of overuse of labor stimulating drugs when it is not effective. Instead, other interventions may be performed. Thus, the proposed technology provides for decreasing risk of the protracted labor.

The expression "indicating" is understood as denoting or signifying. In other words, an indication, or a signal, is provided to a receiver. The receiver may be a human, such as healthcare staff. The receiver may be a device, such as syringe pump or a monitoring device. The indication may be performed in form of an audible signal. The indication may be a visual signal. The indication may be a digital signal provided to a device. The signal may be entered into a database. Preferably the signal may be synchronized with a simultaneously recorded signal associated with a fetus. For example, CTG (cardiotocography) and/or partogram may be used as a signal associated with a fetus. The simultaneously recording of the signals means that the signals may be observed, or managed, relatively to to each other. Preferably, the synchronized signals provide for obtaining a simultaneously recorded signal value, or indication, of combined status for the pregnant woman and the fetus.

The expression "suitability of an action" may be understood as necessity of the action. Thus, if an action is suitable, it may be regarded as necessary. Alternatively, the expression "suitability of an action" may be understood as there is a desired effect associated with the action.

Alternatively, the suitability of an action means that there are no contraindications against the action.

In the context of the technology proposed herein, "administering a drug" is understood as encompassing using methods known in the field, such and subcutaneous, intramuscular, or intravenous injections, to give the drug to a subject, i.e., the pregnant woman. Other suitable methods of administration are by infusion, medical patch or via a venous port. Inhaling a drug or applying it to a mucous membrane is another alternative of administering the drug.

The "labor stimulating drug" is understood as a pharmacologic induction agent. The skilled person recognizes that drug encompasses pharmaceutical. The purpose of the administration may be cervical ripening. The "labor stimulating drug" encompasses drugs for stimulating labor. It is understood that stimulating labor may encompass inducing labor. Stimulating may relate to further stimulating of an already induced labor. The skilled person recognizes that drugs used for inducing and further stimulating of the labor may differ from each other. One example of the labor stimulating drug is synthetic oxytocin. Prostaglandins is another commonly used labor stimulating drug. Nitric oxide donors and mifepristone are other examples of drugs in the area of cervical ripening.

It is understood that the "pregnant woman" in the context is the woman in a condition where a delivery is medically indicated and suitable. It is understood that the expression "pregnant woman" may also encompass a woman having an abortion at a late stage or a woman having a dead fetus which should be delivered vaginally.

The expression "steps" is understood as an action, proceeding, or measure occurring as one in a series.

The expression "determine" is understood as concluding a result. A person skilled in the art would recognise the expression "amniotic fluid lactate concentration value" as the amount of mmol of the urine lactate in one litre of the fluid, preferably amniotic fluid. The determining of the amniotic fluid lactate concentration value may be performed by use of a chemical reaction, such as in an electrochemical electrode. Determining may be brought about as a result by a visual indication, such as colour change of a test strip. The colour change may then be translated into a value by use of a chart or database where associations between the colour and the value are provided. Alternatively, the determining may be accomplished by a current change between the electrodes of an electrochemical chip. Preferably, the precision of the amniotic fluid lactate concentration is 0.15 mmol/l. More preferably, the precision is 0.1 mmol/l. The amniotic fluid lactate concentration value may be measured, or determined, continuously. The determination may be performed intravaginally.

Comparing the amniotic fluid lactate concentration value with the set value means determining whether the amniotic fluid lactate concentration value is below, equal to or above the set value. Preferably, the precision of the set value is 0.15 mmol/l. More preferably, the precision is 0.1 mmol/l. The set value may be a predetermined value set to which the determined amniotic fluid lactate value is compared to manually. The set value and/or the determined amniotic fluid lactate value may be entered manually to a comparison unit where the comparison may be performed. The set value may be hardcoded, or pre-set during manufacturing, in the comparison unit.

The amniotic fluid lactate concentration value at or above 12.0 mmol/l is preferably an amniotic fluid lactate concentration value below 15 mmol/l. For example, the amniotic fluid lactate concentration value above 12.0 mmol/l and below 15 mmol/l may be 13 mmol/l.

The labor stimulating drug may preferably comprise synthetic oxytocin. As described above, oxytocin is recognised as one of possible pharmacologic induction agents. The peptide hormone oxytocin is produced by magnocellular neurosecretory cells within the paraventricular nucleus and the supraoptic nucleus in the hypothalamus. After synthesis it is transported along the axons of these neurons to the neurohypophysis where it is secreted into the bloodstream in pulses. In addition to this global release mechanism, oxytocin is produced locally in specialized cells of the uterus, amnion, chorion and decidua, where it acts as a paracrine signal to influence the behaviour of neighbouring cells. In connection to labor, administration of oxytocin may be used for labor induction, correction of dynamic dystocia or in a situation with no contractions 15 minutes after the birth of first twin.

In a preferred embodiment, an amniotic fluid lactate concentration above 10.1 mmol/l and below 12.9 mmol/l indicates that the rate of administering the labor stimulating drug to the pregnant woman should be decreased. This means that a value of, for example, 10.5 mmol/l is an indication of a need of a decrease of the rate of administration. The rate may be decreased for a certain predetermined period. The decrease may follow a predetermined schedule. The decrease may be linear. The decrease may encompass stopping administering the labor stimulating drug to the pregnant woman.

The method may be repeated after a time period tp. The time period may be 0.5-1 hours, 1-1.5 hours, 1.5 -2 hours, 2-2.5 hours, 2.5-3 hours, 3-3.5 hours, 3.5-4 hours. The time period may be 0.5, 1, 1.5, 2, 2.5, 3, 3.5, or 4 hours. Preferably, the time period is 1 hour. The period tp may be predetermined. The period tp may be scheduled. The period tp may depend on the condition of the pregnant woman, encompassing for example, respiratory rate or estimated pain level. The period tp may depend on the condition of the fetus, encompassing, for example, heart rate of the fetus. It is understood that the time period tp may vary if the method is repeated more than once.

The time period tp may be determined based on the amniotic fluid lactate concentration. For example, the time period tp may be t1 when the amniotic fluid lactate concentration is at or below 10.1 mmol/l. Additionally, the time period tp may be t2 when the amniotic fluid lactate concentration is above 10.1 mmol. Preferably, t2 is smaller than t1. This is advantageous because the time period for the repetition of the method is adapted based on the amniotic fluid lactate concentration. In extension, it means that when the amniotic fluid lactate concentration is above a predetermined level, the method is performed more often. Thus, the condition of the pregnant woman is monitored more often, or frequently, if the amniotic fluid lactate concentration indicates exhaustion of the uterus. In this situation, frequent controls of the amniotic fluid lactate concentration are advantageous as this prevents unnecessary, or undesired, or unsuitable administering of the labor stimulating drug to the pregnant woman.

The amniotic fluid lactate concentration value may be determined by analysing a sample comprising amniotic fluid from the pregnant woman. As described above, if the measured lactate concentration is greater than a predetermined lactate concentration this indicates that amniotic fluid has passed from an amnion of the pregnant woman. The amniotic fluid may be sampled intravaginally. The amniotic fluid may be sampled in an absorption pad. The amniotic fluid may be purified before the measurement.

During pregnancy, the fetus is surrounded and cushioned by a fluid-filled membranous sac called the amniotic sac. Typically, at the beginning of or during labor the membranes will rupture - also known as water breaking. The amniotic fluid lactate concentration value may be determined prior to the rupture of the membranes, for example using a needle passing through the membrane.

The sample may comprise vaginal fluids from the pregnant woman. The sample may contain both amniotic fluid and other vaginal fluids.

The system according to the second aspect of the proposed technology comprises an indication unit. The indication unit may output the indication as a visual indication. The visual indication may be turning on a LED light. For example, if it is suitable to administer a labor stimulating drug to a pregnant woman, a green LED light may be turned on. If it is unsuitable to administer a labor stimulating drug to a pregnant woman, a red LED light may be turned on. Alternatively, or additionally, the indication may be presented on a monitor. For example, the indication may be in form of a text and/or a sign. The monitor may, for example, be a CRT (Cathode Ray tube), LCD (Liquid Crystal Display), LED (Liquid Emitting Diode), or OLED (Organic Light Emitting Diode) monitor.

This is advantageous as it provides for a straight-forward use of the system. This means that it requires less education in using the system. Moreover, the visual indication is especially useful in stressful situations as during labor.

The system may further comprise an administration device configured to administer the labor stimulating drug. The administration device may be an infusion pump. The administration device may further be operably connected to the indication unit. If the administration device is an infusion pump, it may be connected to an infusion packaging such is prefilled syringe such that the device may force the drug out of the syringe into the connection coupled to the pregnant woman.

The administration device may be configured to a. administer the labor stimulating drug when the amniotic fluid lactate concentration value is at or below 10.1 mmol/l. This means that the administration device may be programmed to start or continue administering the labor stimulating drug when the amniotic fluid lactate concentration value is at or below 10.1 mmol/l. Alternatively, or additionally, the administration device may be manually controlled such by a knob, or a lever.

The administration device may be configured to b. not administer the labor stimulating drug when the amniotic fluid lactate concentration value is at or above 12.0 mmol/l. This means that the administration device may be programmed to discontinue, or cancel, or not start administering the labor stimulating drug when the amniotic fluid lactate concentration value is at or above 12.0 mmol/l. This is advantageous due to increased level of automation. This provides for less interventions by the healthcare stuff which make the procedure more time- and cost-efficient. Moreover, the risk for mistakes is avoided.

The sensor may be configured to determine the amniotic fluid lactate concentration value by analysing a sample comprising amniotic fluid from the pregnant woman. The sensor may be an electrochemical sensor. The sensor may be coated with a biochemical composition for recognizing the substance lactate in the sample. The sensor may send a signal to a receiving device. The receiving device may convert the signal to a lactate value. Alternatively, the signal conversion may be integrated in the sensor.

The sensor may comprise an absorbent body for absorbing amniotic fluid, alone or mixed with vaginal fluids, from the pregnant woman. The absorbent body may for example be a tampon, a sanitary pad or an underlay. It is understood that the absorbent body comprises absorbent material, such as sodium polyacrylate, which absorbs amniotic fluid and vaginal fluids from the pregnant woman. Preferably, the absorbent body is positioned outside the body of the pregnant woman. It is understood that the absorbent body may be replaced with a new one when needed. The absorbent body may be disposable such as being suitable for one time usage. The absorbent body may be washable and configured to be reused.

The absorbent body preferably further comprises an additional sensor for detecting the presence of substances or compounds, in particular urine, that may interfere with determining the amniotic fluid lactate concentration value. The sensor and/or comparison unit may be configured to apply a correction to the determined amniotic fluid lactate concentration value if the additional sensor detects the presence of substances or compounds that interfere with determining the amniotic fluid lactate concentration value. Alternatively, or additionally the indication unit may be configured to receive a signal from the additional sensor and provide an indication if the additional sensor detects the presence of substances or compounds that interfere with determining the amniotic fluid lactate concentration value.

The presence of the absorbent body provides for analysis of the amniotic fluid lactate concentration value without additional sampling. Instead, the amniotic and/or vaginal fluids are collected in the absorbent body and analysed. This is both time- and cost efficient. Additionally, it is more convenient to the pregnant women as no additional interventions are required.

The sensor may be configured to be attached to the fetus. The attachment may be performed by technologies known in the field for attaching other sensors to a fetus, such as by spiral.

For example, the sensor may be comprised in a fetal scalp electrode. This is advantageous because such fetal scalp electrodes may provide information regarding both the fetus and the pregnant woman. Only one intervention, namely attaching the fetus scalp electrode to the fetus, is required to receive the data. This makes the procedure both time- and cost-efficient. Moreover, as the information regarding both the fetus and the pregnant woman is provided, it is easier to follow the progress of the labor in relation to the status of the fetus. Therefore, it is easier to make a decision regarding necessary further interventions.

Preferably, the sensor is positioned so as to sample amniotic fluid in the uterus. This means that the sensor may be positioned so as to receive the amniotic fluid.

It is understood that the sensor may be attached to other body part of the fetus than the scalp.

The sensor may comprise a catheter for collecting the sample comprising amniotic fluid from the pregnant woman. It is understood that the catheter comprises an opening for receiving or collecting the sample. The opening is positioned on a proximal end of the catheter. The opening of the catheter may be positioned intravaginally or in the uterus. The catheter may partially extend out of the body of the pregnant woman in order to lead amniotic fluid from the proximal end to a distal end at which it can be collected, or at which end the sensor may determine the amniotic fluid lactate concentration value. The extending part may be configured to be attached to the body of the pregnant woman. It is understood that only a part of the catheter is attached to the pregnant woman, and that the attaching may allow limited movement of the catheter related to the body of the pregnant woman. For example, the catheter may be attached to a thigh or a leg of the pregnant woman. The catheter may be attached to the abdomen of the pregnant woman. The attachment may be provided by medical tape. Alternatively or additionally, the catheter may be attached by an attachment unit. Preferably, the attachment unit is configured for being attached to the pregnant woman, thereby attaching the catheter, by means of an elastic belt or ribbon, or by a belt or strap closable by a hook and loop closure.

The catheter may be a soft catheter. This provides for flexibility of the position of the pregnant woman during labor. The catheter may be manufactured of silicone rubber, nylon, polyurethane, polyethylene terephthalate (PET), latex, or thermoplastic elastomers. The catheter may be connected to the sensor at the distal end. The connection may be permanent. Alternatively, the sensor may be attached to the catheter by, for example, Luer lock. This provides for reusing the sensor.

Alternatively, a solid elongated probe may be used instead of the catheter, in which case the sensor is placed on the tip of the elongated probe. The solid elongated probe may be attached to the pregnant woman as described above for the catheter.

A third aspect of the technology proposed herein relates to the sensor as described above in relation to the system according to the second aspect of the technology proposed herein. The sensor is preferably configured to determine the amniotic fluid lactate concentration value by analysing a sample comprising amniotic fluid from the pregnant woman as described above. Further, the sensor preferably comprises an absorbent body for absorbing amniotic fluid, alone or mixed with vaginal fluids, from the pregnant woman as described above. Alternatively, the sensor may be configured to be attached to the fetus as described above. Alternatively, the sensor may comprise a catheter configured to be partially attached to the body of the pregnant woman.

A fourth aspect of the technology proposed herein relates to a method of administering a labor stimulating drug to a pregnant woman, comprising the steps of:
i. performing the method according to first aspect of the technology proposed herein and obtaining an indication that the labor stimulating drug is suitable to be administered to the pregnant woman, and
ii. administering the labor stimulating drug to the pregnant woman.

A fifth aspect of the technology proposed herein relates to a method of treating a pregnant woman, comprising the steps of:
i. performing the method according to first aspect of the technology proposed herein and obtaining an indication that the labor stimulating drug is not suitable to be administered to the pregnant woman, and
ii. not administering the labor stimulating drug to the pregnant woman.

### Brief description of the drawings and detailed description

A more complete understanding of the abovementioned and other features and advantages of the technology will be apparent from the following detailed description of preferred embodiments in conjunction with the appended drawings, wherein:
- Fig. 1: shows a flowsheet of the method according to the first aspect of the technology proposed herein.
- Fig. 2A-B: shows an embodiment of the system according to the second aspect of the technology proposed herein.
- Fig. 3A-3E: show different embodiments of the sensor comprised by the system according to the second aspect of the technology proposed herein.

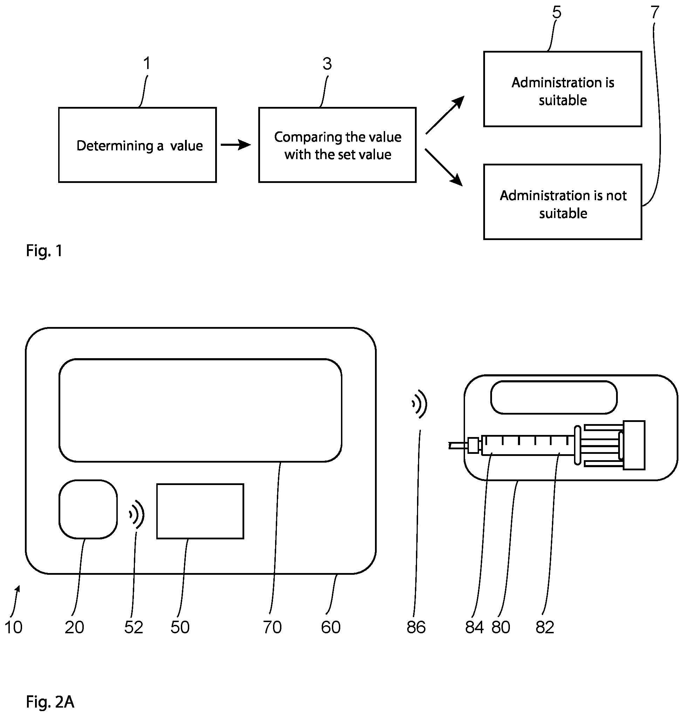

Fig. 1 shows a flowsheet of the method according to the first aspect of the technology proposed herein. The method starts with a first step 1 of determining an amniotic fluid lactate concentration value. This is followed by a second step 3 of comparing the amniotic fluid lactate concentration value determined in the first step 1 with the set value of 10.1 mmol/l and the set value 12.0 mmol/l, whereby: a. an amniotic fluid lactate concentration value at or below 10.1 mmol/l indicates 5 that the labor stimulating drug is suitable to be administered to the pregnant woman, and b. an amniotic fluid lactate concentration value at or above 12.0 mmol/l indicates 7 that the labor stimulating drug is not suitable to be administered to the pregnant woman.

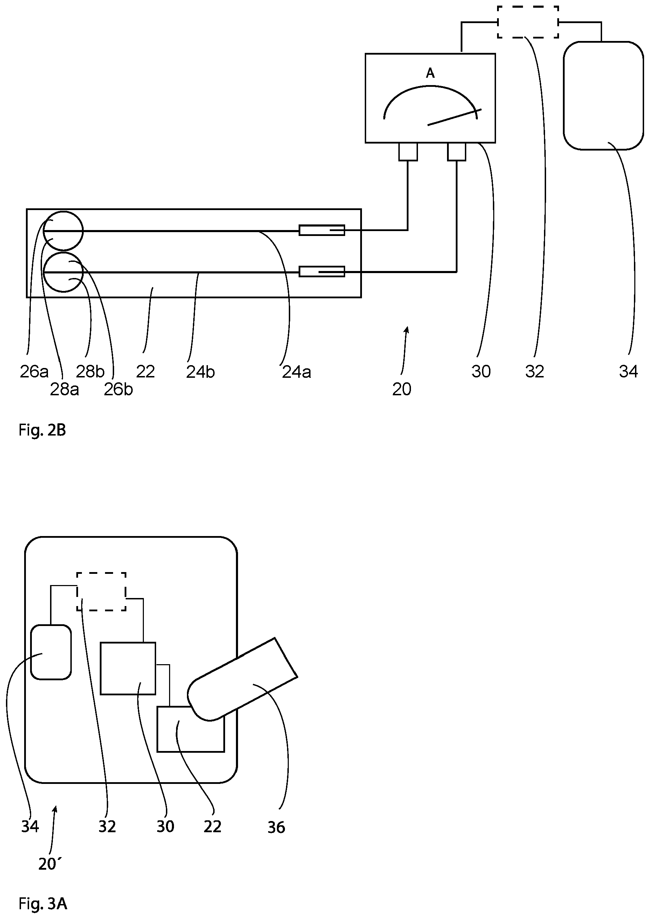

Fig. 2 shows an embodiment of the system according to the second aspect of the technology proposed herein. The system 10 comprises a sensor 20 configured to determine an amniotic fluid lactate concentration value, and a comparison unit 50 configured to compare the amniotic fluid lactate concentration value with the set value of 10.1 mmol/l and the set value 12.0 mmol/l. The sensor 20 and the comparison unit 50 may be separate from each other, in which case the amniotic fluid lactate concentration value is sent via a wired or wireless connection 52 to the comparison unit 50. Alternatively, as here, the sensor 20 and the comparison unit 50 are provided in a common housing 60 for convenience. The system 10 further comprises an indication unit, here represented by screen 70, on which a. an indication that the labor stimulating drug should be administered to the pregnant woman when the amniotic fluid lactate concentration value is at or below 10.1 mmol/l can be outputted, and alternatively, b. an indication that the labor stimulating drug should not be administered to the pregnant woman when the amniotic fluid lactate concentration value is at or above 12.0 mmol/l can be outputted. Also preferably comprised by the system 10 is an administration device here represented by syringe pump 80 connected to a source, here represented by syringe 82, of labor stimulating drug 84. The administration device 80 is operably connected to the indication unit 70 or comparison unit 50 via a wired or wireless connection 86 and is configured to a. administer the labor stimulating drug 84 when the amniotic fluid lactate concentration value is at or below 10.1 mmol/l, and b. not administer the labor stimulating drug 84 when the amniotic fluid lactate concentration value is at or above 12.0 mmol/l.

Fig. 2B shows the sensor in more detail. Preferably the sensor 20 comprises an enzyme, preferably lactate oxidase (LOX), to convert lactate in one or more reactions eventually producing one or more electrodes which can be detected and read out as current. In one preferred embodiment the sensor 20 is an electrochemical sensor. The electrochemical sensor may for example comprise lactate oxidase (LOX) and a mediator such as tetrathiafulvalene. In this case the following reactions (1)-(3) occur in the sensor:

Lactate + LOX(FAD) ↔ Pyruvate + LOX(FADH2) (1)

LOX(FADH2) + 2TTF+ ↔ LOX(FAD) + 2TTF + 2H+ (2)

2TTF ↔ 2TTF+ +2e- (3)

LOX refers to lactate oxidase enzyme, FAD refers to Flavin adenine dinucleotide of lactate oxidase, and TTF refers to tetrathiafulvalene. In reaction (1) lactate reacts with LOX to produce pyruvate and reduce the FAD of LOX. In reaction (2) the reduced version of LOX reacts with oxidized TTF to oxidize LOX and reduce TTF. In reaction (3) TTF is oxidized to produce electrons, which result in a current between electrodes over which a voltage is applied. The purpose of the mediator TTF is to decrease the operation voltage of the sensor so as to avoid oxidizing other compounds that might be present in the sample.

The sensor 20 thus preferably comprises an electrochemical sensing element 22 comprising a working electrodes 24a and a reference electrode 24b. Lactate oxidase (LOX) 26a and a mediator 28a such as tetrathiafulvalene as described above are provided on the working electrode 24a. The reference electrode may use a silver 26b / silver chloride 28b setup. The electrochemical sensing element 22 is brought into contact with lactate, a voltage is applied over the electrodes 24a and 24b, lactate present is converted into pyruvate, and the reactions (1)-(3) ultimately leads to the obtainment of electrons, which, under the voltage applied, result in a current running between the electrodes 24a and 24b, which current is measured using ammeter 30, optionally converted into a digital value using an analog-digital converter 32 and compared to a calibration curve associating current values to lactate concentrations in an evaluation unit 34 to produce the amniotic fluid lactate concentration value. Depending on implementation, one or more of the ammeter 30, the analog-digital converter 32 and the evaluation unit 34 may be provided separate from the electrochemical sensing element 22, for example in the common housing 60 or as part of the comparison unit 50.

Fig. 3A-3C show different embodiments of the sensor comprised by the system according to the second aspect of the technology proposed herein.

Fig. 3A shows a first alternative embodiment of a sensor 20' configured to determine the amniotic fluid lactate concentration value by analysing a sample comprising amniotic fluid from a pregnant woman. The sensor 20' comprises a receptacle 36 for receiving and holding a sample comprising amniotic fluid. The sensor 20' further comprises the electrochemical sensing element 22, ammeter 30, the optional analog-digital converter 32, and the evaluation unit 34 as described above. The sensor 20' is integrated in the common housing 60 and the receptacle 36 is removable from the sensor 20' for simplifying providing the sample in the receptacle 36.

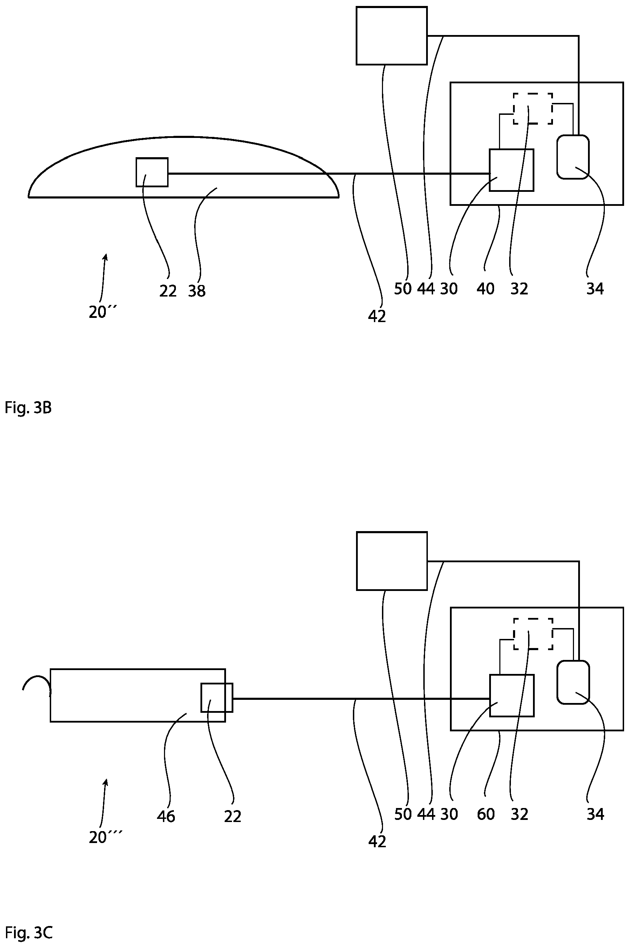

Fig. 3B shows a second alternative embodiment of a sensor 20" comprising an absorbent body 38 for absorbing vaginal fluids and amniotic fluid from the pregnant woman. The absorbent body 38 is shaped as a generally planar pad so as to be placeable on a labour delivery room bed or obstetric delivery table in a suitable position for receiving and absorbing fluids. In the sensor 20" the electrochemical sensing element 22 is provided inside the absorbent body 38, e.g., by being laminated between sheets of absorbent material making up the absorbent body 38. The ammeter 30, the optional analog-digital converter 32, and the evaluation unit 34 are preferably provided separate from the electrochemical sensing element 22, such as in the common housing 60 or in a separate module 40. The electrochemical sensing element 22 is preferably provided with a wired connection 42 to the ammeter 30. When the ammeter 30, the optional analog-digital converter 32, and the evaluation unit 34 are provided in the separate module 40, a wireless or wired connection 44 is used to transfer the amniotic fluid lactate concentration value to the comparison unit 50.

Once the absorbent body 38 with electrochemical sensing element 22 has been used to receive and absorb vaginal and amniotic fluids in order to measure the amniotic fluid lactate concentration value, it is discarded and replaced with a new absorbent body 38 with electrochemical sensing element 22. Alternatively, only the absorbent body 38 is replaced and the electrochemical sensing element 22 is washed before being inserted into a new absorbent body 38.

Fig. 3C shows a third alternative embodiment of a sensor 20‴ wherein the sensor 20‴ is configured to be attached to the fetus. In the sensor 20‴ the electrochemical sensing element 22 is provided in a connection element 46 comprising a scalp electrode which is attachable to the fetus. Sensor 20‴ may thus be implemented as part of a fetal scalp electrode as used to measure fetal heart rate. By providing the electrochemical sensing element 22 in the connection element 46 the electrochemical sensing element 22 is positioned so as to sample amniotic fluid in the uterus. Further, by monitoring the heart rate using the fetal scalp electrode and measuring the amniotic fluid lactate concentration value using the sensor 20‴, the labor status of the pregnant woman, and the status of the fetus can be monitored. In the sensor 20‴, the ammeter 30, the optional analog-digital converter 32, and the evaluation unit 34 are preferably provided separate from the electrochemical sensing element 22, such as in the common housing 60 or in the separate module 40 shown in Fig. 3B. Further, the electrochemical sensing element 22 is preferably provided with a wired connection 42 to the ammeter 30. When the ammeter 30, the optional analog-digital converter 32, and the evaluation unit 34 are provided in the separate module 40, a wireless or wired connection 44 is used to transfer the amniotic fluid lactate concentration value to the comparison unit 50.

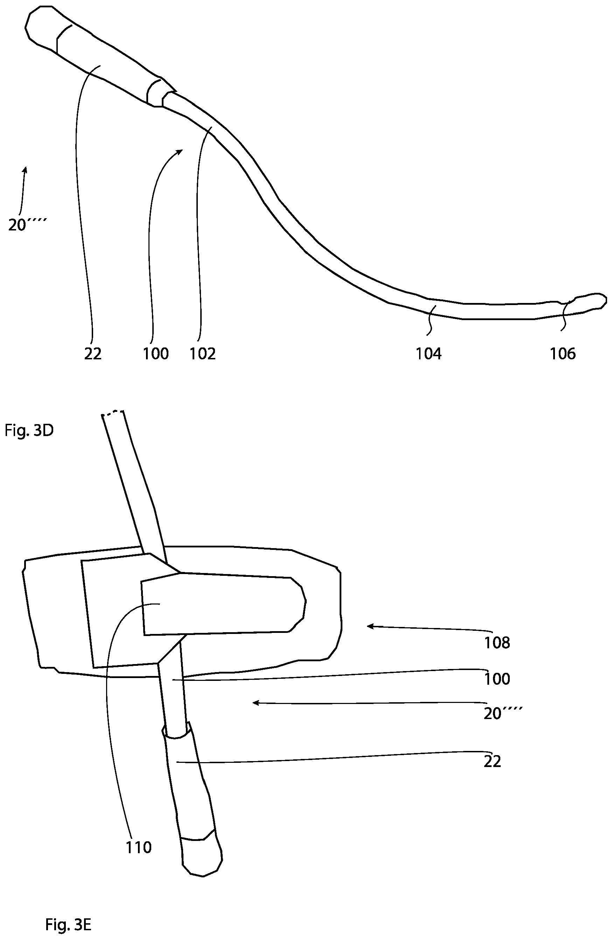

Fig. 3D and 3E show a fourth alternative embodiment of a sensor 20"" wherein the sensor 20"" comprises a catheter 100 which is configured to be attached to the body of the pregnant woman. In the sensor 20‴ the electrochemical sensing element 22 is attached to a distal end 102 of the catheter. A proximal end 104 of the catheter 100 is intended to be inserted into the body of the pregnant woman for collecting a sample. The catheter has an opening 106 at the distal end. The sample is collected via the opening 106 and led to the distal end 102 where it measured by the sensing element 22.

The catheter may be attached to the thigh of the pregnant woman by use of the attaching unit 108. For attaching, the attaching unit 108 is fitted around a thigh of the pregnant woman by tightening and the catheter is inserted into and fixed in the attaching unit 108 by hook and loop closure 110.

### Feasible modifications of the technology proposed herein

The technology proposed herein is not limited to the embodiments described above and shown in the drawings, which primarily have an illustrative and exemplifying purpose. This patent application is intended to cover all adjustments and variants of the preferred embodiments described herein, thus the present invention is defined by the wording of the appended claims and the equivalents thereof. Thus, the equipment may be modified in all kinds of ways within the scope of the appended claims.

It shall also be pointed out that all information about/concerning terms such as above, under, upper, lower, etc., shall be interpreted/read having the equipment oriented according to the figures, having the drawings oriented such that the references can be properly read. Thus, such terms only indicate mutual relations in the shown embodiments, which relations may be changed if the inventive equipment is provided with another structure/design.

It shall also be pointed out that even though it is not explicitly stated that features from a specific embodiment may be combined with features from another embodiment, the combination shall be considered obvious, if the combination is possible. Throughout this specification and the claims which follows, unless the context requires otherwise, the word "comprise", and variations such as "comprises" or "comprising", will be understood to imply the inclusion of a stated integer or steps or group of integers or steps but not the exclusion of any other integer or step or group of integers or steps.

## Claims

1. A method for indicating the suitability of administering a labor stimulating drug to a pregnant woman comprising the steps of:
i. determining an amniotic fluid lactate concentration value,
ii. comparing the amniotic fluid lactate concentration value with the set value of 10.1 mmol/l and the set value 12.0 mmol/l, whereby:
a. an amniotic fluid lactate concentration value at or below 10.1 mmol/l indicates that the labor stimulating drug is suitable to be administered to the pregnant woman, and
b. an amniotic fluid lactate concentration value at or above 12.0 mmol/l indicates that the labor stimulating drug is not suitable to be administered to the pregnant woman.

2. The method according to claim 1, wherein the amniotic fluid lactate concentration value at or above 12.0 mmol/l is an amniotic fluid lactate concentration value below 15 mmol/l.

3. The method according to any preceding claim, wherein the labor stimulating drug comprises oxytocin.

4. The method according to any preceding claim, whereby:
c. an amniotic fluid lactate concentration above 10.1 mmol/l and below 12.9 mmol/l indicates that the rate of administering the labor stimulating drug to the pregnant woman should be decreased.

5. The method according to any preceding claim, wherein the method is repeated after a time period tp.

6. The method according to claim 5, wherein the time period tp is determined based on the amniotic fluid lactate concentration such that:
d. the time period tp is t1 when the amniotic fluid lactate concentration is at or below 10.1 mmol/l, and
e. the time period tp is t2 when the amniotic fluid lactate concentration is above 10.1 mmol, and
wherein t2 is smaller than t1.

7. The method according to any preceding claims, wherein the amniotic fluid lactate concentration value is determined by analysing a sample comprising amniotic fluid from the pregnant woman.

8. The method according to any preceding claim, wherein the sample comprises vaginal fluids from the pregnant woman.

9. A system for indicating the suitability of administering a labor stimulating drug to a pregnant woman comprising:
i. a sensor configured to determine an amniotic fluid lactate concentration value,
ii. a comparison unit configured to compare the amniotic fluid lactate concentration value with the set value of 10.1 mmol/l and the set value 12.0 mmol/l,
iii. an indication unit configured to output:
a. an indication that the labor stimulating drug is suitable to be administered to the pregnant woman when the amniotic fluid lactate concentration value is at or below 10.1 mmol/l, and
b. an indication that the labor stimulating drug is suitable to be administered to the pregnant woman when the amniotic fluid lactate concentration value is at or above 12.0 mmol/l.

10. The system according to claim 9, wherein the indication outputted by the indication unit comprises a visual indication.

11. The system according to claim 10, further comprising: iv. an administration device configured to administer the labor stimulating drug, the administration device further being operably connected to the indication unit and configured to:
a. administer the labor stimulating drug when the amniotic fluid lactate concentration value is at or below 10.1 mmol/l, and
b. not administer the labor stimulating drug when the amniotic fluid lactate concentration value is at or above 12.0 mmol/l.

12. The system according to any of the claims 9-11, wherein the sensor is configured to determine the amniotic fluid lactate concentration value by analysing a sample comprising amniotic fluid from the pregnant woman.

13. The system according to any of the claims 9-12, wherein the sensor comprises an absorbent body for absorbing amniotic fluid, alone or mixed with vaginal fluids, from the pregnant woman.

14. The system according to any of the claims 9-13, wherein the sensor is configured to be attached to the fetus.

15. The system according to any of the claims 9-14, wherein the sensor comprises a catheter for collecting the sample, wherein preferably the catheter is configured to be partially attached to the body of the pregnant woman.
